# DEMANDE DE BREVET EUROPEEN

(11) **EP 0 953 338 A1**
(43) Date de publication de la demande: **03.11.1999**
(21) Numéro de dépôt: 98118997.0
(22) Date de dépôt: 15.12.1994
(51) Int. Cl.: A61K 7/48, A61K 7/06, B65D 47/44, B65B 31/00, B65D 83/14, B65D 81/26, B65D 81/30

(54) **Dispositif de conditionnement d'un produit**

(30) Priorité: 30.12.1993 FR 9315862
(62) Demande divisionnaire de: 94402619.4
(71) Demandeur: L'OREAL, 75008 Paris (FR)
(72) Inventeur: Collin, Nathalie, 92330 Sceaux (FR); Quemin, Eric, 93290 Tremblay en France (FR)
(74) Mandataire: Tezier Herman, Béatrice

(57) **Abrégé**

L'invention se rapporte à un dispositif de conditionnement d'un produit, caractérisé par le fait qu'il est muni d'un dispositif de distribution tel que le produit n'est pas mis en contact avec l'environnement, que les parois le constituant sont imperméables aux gaz et à la lumière ultraviolette visible, leur surface en contact avec le produit étant non métallique, et qu'il contient en outre un dispositif de capture de l'oxygène.

Ce dispositif est parfaitement adapté à des émulsions eau dans huile contenant, de manière stable, du rétinol, destinées en particulier aux domaines cosmétique et/ou dermatologique.

## Description

La présente invention a pour objet un dispositif de conditionnement parfaitement adapté à des émulsions eau dans huile contenant, de manière stable, du rétinol, destinées en particulier aux domaines cosmétique et/ou dermatologique.

Le rétinol est connu pour ses effets bénéfiques sur la peau, notamment en application topique.

Le rétinol est, depuis longtemps, utilisé dans le traitement de l'acné. Mais c'est dans le domaine de la correction des dommages induits soit par l'âge, soit par une exposition au soleil trop intensive, que le rétinol s'est révélé extrêmement actif.

Ainsi, les effets du rétinol sur la différenciation cellulaire permettent d'envisager, entre autre, son utilisation pour lutter efficacement contre l'apparition des rides et ridules, contre la sécheresse cutanée, la rugosité et/ou la rigidité de la peau. Son activité dans la régénération des tissus en fait un composé important dans la cicatrisation. Une application répétée de compositions cosmétiques contenant du rétinol permet, entre autre, d'effacer les rides, de lisser la peau, de réparer les petites déchirures de l'épiderme.

Du fait de ces effets bénéfiques, on cherche depuis très longtemps, à formuler le rétinol dans des compositions cosmétiquement acceptables, sous forme stable pendant au moins plusieurs mois à température ambiante.

Des émulsions utilisables en cosmétique contenant du rétinol sont décrites, en particulier dans le document US-A-4,826,828 et dans le document WO-A-93/00085.

Dans le document US-A-4,826,828, il s'agit d'émulsions eau dans huile contenant le rétinol, une silicone volatile, ainsi qu'un solvant du rétinol et de la silicone volatile. Le solvant préféré est l'éthanol. Or, la demanderesse a montré récemment que le rétinol se dégrade en présence d'éthanol. Pour obtenir l'émulsion, il est enseigné dans ce document US-A-4,826,828 de préparer une solution contenant le rétinol à mélanger au moment de l'emploi, pour éviter toutes dégradations de celui-ci, à une émulsion eau dans huile. En outre, l'emploi d'antioxydant et d'agent chélateur de métaux dans la phase aqueuse est indiqué comme essentiel.

La stabilité de telles compositions, comme l'indique le détenteur du brevet US-A-4,826,828 sur les emballages de ses produits Bioadvance et Bioadvance 2000 décrits dans ce brevet, n'excède pas un mois. Ainsi, la stabilité du rétinol dans ce type de compositions est insuffisante au regard d'une utilisation prolongée, ce qui nécessite un réapprovisionnement rapide, donc coûteux.

Dans le document WO-A-93/00085, il est proposé une stabilisation du rétinol dans les compositions cosmétiques par adjonction à celles-ci d'un complexe stabilisant, comprenant associés, un antioxydant et un agent chélateur d'ions métalliques. Si, toutefois, la stabilité du rétinol semble améliorée dans de telles compositions (60 % du rétinol étant encore présent dans la composition après trois mois de conservation à 40°C), il n'en demeure pas moins que la stabilité relative du rétinol n'est due qu'à la présence, dans la composition, d'une quantité importante d'antioxydants et d'agents chélateurs stabilisants.

De nombreuses recherches ont été menées afin de diminuer au maximum, voir d'éliminer, la présence de tels stabilisants dans les compositions cosmétiques contenant du rétinol, tout en conservant à celui-ci une stabilité dans la composition, acceptable au regard de ses effets et en relation avec une utilisation prolongée de la composition.

De manière surprenante et inattendue, la demanderesse a découvert qu'il est possible de formuler des émulsions eau dans huile, stables dans le temps, contenant du rétinol, destinées à une utilisation topique, par un choix approprié des différents constituants de l'émulsion dans le but de conférer à celle-ci des propriétés physico-chimiques particulières.

L'émulsion de l'invention est une émulsion eau dans huile contenant du rétinol, une phase grasse et une phase aqueuse, caractérisée par le fait que la phase grasse contient, au moins, un solvant organique du rétinol liquide à température ambiante et que le pH de la phase aqueuse est basique, de façon à stabiliser le rétinol, au moins 2 mois. Les essais réalisés avec une émulsion selon l'invention ont montré une stabilité du rétinol nettement améliorée par rapport à celle de l'art antérieur.

L'émulsion selon l'invention a l'avantage de pouvoir contenir encore 82 % de rétinol actif après conservation à 45°C pendant 2 mois.

La présente invention a pour objet un conditionnement approprié à l'émulsion décrite ci-dessus.

Ce conditionnement se caractérise par le fait qu'il est muni d'un dispositif de distribution tel que le produit qu'il contient ne soit pas mis en contact avec l'environnement, qu'il contienne en outre un dispositif de capture de l'oxygène et que ses parois soient imperméables au gaz et à la lumière ultraviolette visible.

Le dispositif de distribution peut être constitué par une valve ou une pompe sans reprise d'air, tel que le dispositif décrit dans le document FR-A-2,666,308 ou dans le document FR-A-2,658,739.

Dans un mode particulier de l'invention, le dispositif de capture de l'oxygène est isolé du produit contenu dans le conditionnement par une membrane perméable au gaz et imperméable aux liquides notamment ceux entrant dans la composition de l'émulsion selon l'invention.

La membrane perméable au gaz peut être, par exemple, celle définie dans le document FR-A-2,671,055.

Comme dispositif de capture de l'oxygène, on peut utiliser tout moyen capable d'absorber l'oxygène sans relargage. On peut citer par exemple ceux vendus par la société ATCO sous le nom de "absorbeur d'oxygène LH50" ou "absorbeur d'oxygène LH100, LH 500 et autres".

Le dispositif de capture de l'oxygène isolé par la membrane peut être, dans le dispositif de conditionnement, solidaire des parois ou non solidaire de ces parois et noyé dans la composition.

L'émulsion utilisée selon l'invention est parfaitement bien adaptée à une utilisation cosmétique et/ou dermatologique.

Les proportions des différents constituants de l'émulsion selon l'invention sont celles classiquement utilisées dans le domaine cosmétique ou dermatologique et sont fonction de l'application spécifique envisagée.

En outre, dans une réalisation préférentielle, l'émulsion selon l'invention est exempte de tout agent chélateur d'ions métalliques et éventuellement d'agent antioxydant.

En particulier, la phase grasse de l'émulsion selon l'invention peut représenter de 10 % à 50 % du poids total de l'émulsion et de préférence de 20 % à 35 %.

Par ailleurs, la phase aqueuse de l'émulsion selon l'invention peut représenter de 50 % à 90 % du poids total de l'émulsion et de préférence de 65 % à 80 %.

De manière générale, le rétinol est présent dans l'émulsion selon l'invention dans une proportion allant de 0,001 % à 10 %, et de manière préférentielle de 0,01 % à 1 % du poids total de l'émulsion.

Le rétinol utilisé dans l'émulsion selon l'invention peut être du rétinol dans l'une quelconque de ses conformations et en particulier celui sous la forme tout-trans, tel que celui vendu par la société Fluka sous la dénomination "all-trans-rétinol". Le rétinol tout-trans a la particularité de présenter une activité de rétinoïde et une innocuité supérieure aux autres rétinoïdes.

Le solvant organique du rétinol peut représenter de 0,01 % à 30 % du poids total de l'émulsion selon l'invention, et de préférence de 0,1 % à 10 %.

Le solvant organique liquide à température ambiante (à 20°C par exemple) utilisé dans l'invention est n'importe quel solvant organique susceptible de dissoudre le rétinol tout en lui conservant une bonne stabilité. En particulier, ce solvant est choisi dans le groupe constitué par les alcools gras aliphatiques à chaîne ramifiée en C₁₆-C₂₀, les diesters d'acide dicarboxylique en C₆-C₁₄ et d'alcool isopropylique, les triglycérides d'acide gras en C₆-C₁₈ et leurs mélanges.

Les solvants du type alcools gras à chaîne droite ou ramifiée, alcoxylés, détruisent la stabilité de l'émulsion selon l'invention.

Parmi les alcools gras aliphatiques en C₁₆-C₂₀ utilisables dans l'invention, on peut citer, l'hexyl-2-décanol tel que celui vendu par la société Condea sous la dénomination "Isofol 16", l'octyldodécanol tel que celui vendu par la société Henkel sous la dénomination "Eutanol G", et l'alcool isostéarylique tel que celui vendu par la société Sherex sous la dénomination de "Adol 66".

Parmi les diesters dicarboxyliques en C₆-C₁₄ et d'alcool isopropylique utilisables dans l'invention, on peut citer l'adipate de di-isopropyle tel que celui vendu par la société ISP sous la dénomination de "Ceraphyl 230".

Parmi les triglycérides d'acides gras en C₆-C₁₈, selon l'invention, on préfère utiliser des triglycérides mixtes des acides capriques/capryliques tels que ceux vendus par la société Hüls France sous la dénomination de "Miglyol 812".

Dans une réalisation particulière de l'invention, il est possible d'utiliser le mélange de deux ou plusieurs de ces solvants du rétinol.

D'autres constituants conventionnels des compositions cosmétiques, pharmaceutiques ou vétérinaires peuvent être introduits dans les émulsions selon l'invention. La nature de ces ingrédients et leur proportion doit être compatible avec la stabilité recherchée du rétinol dans les émulsions selon l'invention.

La phase grasse de l'émulsion selon l'invention peut contenir un émulsionnant capable de former une émulsion eau dans huile et supportant des pH basiques. Cet émulsionnant est en particulier un émulsionnant siliconé utilisé dans une proportion de 0,5 % à 10 % et de préférence de 1 % à 6 % du poids total de l'émulsion. Parmi les émulsionnants pouvant entrer dans la composition de la phase grasse, on peut citer les alkyl diméthicone copolyols en C₁₀-C₂₂ polyoxyéthylénés tels que le cétyl diméthicone copolyol ou "Abil EM-90" de la société Goldschmidt, ou le lauryl diméthicone copolyol polyoxyéthyléné et polyoxypropyléné comme le Q2-5200 de la société Dow Corning, et un mélange des deux.

On peut aussi utiliser les diméthicone copolyols polyoxyéthylénés tel que le SP 1228 de General Electric et également le Q2-3225 C de la société Dow Corning.

La phase grasse peut également contenir une huile complémentaire inerte vis-à-vis du rétinol, différente de l'émulsionnant que l'on choisit de préférence parmi les huiles minérales ou siliconées.

Parmi les huiles minérales utilisables selon l'invention, on peut citer, entre autre, l'isohexadécane, la paraffine, l'isoparaffine, la vaseline.

Parmi les huiles siliconées utilisables dans l'invention, on peut citer, entre autre, les diméthicones, les diméthiconols, les cyclométhicones telles que la cyclopentadiméthylsiloxane (ou cyclométhicone D5) ou la cyclohexadiméthylsiloxane (ou cyclométhicone D6) ou les alkyl diméthicones et un mélange de certains de ces composés tel que la "gomme Q2-1401" de Dow Corning.

La phase aqueuse de l'émulsion selon l'invention a un pH basique supérieur à 8 et de préférence allant de 8,5 à 9,5. Le pH de la phase aqueuse est obtenu par adjonction de toute base compatible avec la stabilité du rétinol dans l'émulsion selon l'invention et en quantité appropriée pour obtenir le pH recherché de la phase aqueuse. En pratique, on utilisera la base à raison d'une proportion allant de 0,05 % à 0,5 % du poids total de l'émulsion.

Parmi les bases utilisées dans l'invention, on peut citer par exemple la triéthanolamine, l'aminométhylpropanol, l'aminométhylpropanediol, le tris ou aminométhylpropanetriol, la soude, l'arginine, la lysine.

L'émulsion selon l'invention peut contenir, en outre, des additifs hydrophiles ou lipophiles classiquement utilisés dans les compositions cosmétiques ou dermatologiques.

Parmi les additifs hydrophiles utilisés dans l'émulsion selon l'invention, on peut citer les hydratants comme les polyols (le glycérol) et les glycols.

Parmi les additifs lipophiles, on peut citer les gélifiants comme le gel de bentone, par exemple celui vendu sous le nom de "Simagel SI 345" par la société Biophil, les cires de polyéthylène, comme la cire de polyéthylène AC 617 de la société Allied Chemical.

L'émulsion selon l'invention contient avantageusement des sels stabilisants d'origine non métallique dans une proportion, par exemple, de 0,3 % à 2 % du poids total de l'émulsion tels, que le chlorure de sodium (NaCl), le chlorure de potassium (KCl).

La préparation de l'émulsion de l'invention est effectuée en atmosphère inerte (azote ou gaz rare tel que l'Argon) exempte de tout oxygène et en présence de lumière inactinique telle que celle d'une lampe à vapeur de sodium.

On va maintenant décrire un procédé général de préparation des émulsions selon l'invention. Dans un premier temps, les phases grasse (A) et aqueuse (B) constituant l'émulsion sont préparées indépendamment, puis la phase aqueuse (B) est incorporée à la phase grasse (A) sous agitation au Moritz, moyenne vitesse, à température ambiante, dans les conditions normales de préparation de toutes émulsions eau dans huile. Le mélange ainsi formé constitue le support émulsionné dans lequel on va incorporer le rétinol.

Sous une tente à azote, à température ambiante, en salle éclairée par une lampe à vapeur de sodium, on fait dégazer l'ensemble des éléments de l'émulsion selon l'invention afin d'éliminer toute trace d'oxygène et de remplacer celle-ci par de l'azote. Ce dégazage s'effectue pendant au moins deux heures. Quand toute trace d'oxygène est évacuée (on peut vérifier ceci par utilisation d'un oxymètre), on pèse le rétinol et son solvant, et on les mélange sous agitation magnétique jusqu'à solubilisation totale pour former une phase (C). Puis on incorpore cette phase (C) à l'émulsion par agitation à la pâle pour une bonne homogénéisation. Une fois terminée, l'émulsion est incorporée toujours en atmosphère inerte d'azote et sous lumière de vapeur de sodium dans un conditionnement tel que défini ci-dessus.

Nous allons maintenant, de manière non limitative et à titre d'exemple, donner des compositions de diverses émulsions réalisées selon l'invention.

### Exemple 1 - Crème contour des veux

| | | |
|---|---|---|
| A- | Abil EM-90 | 2 % |
| | Cyclométhicone D5 | 7 % |
| | Simagel Si 345 | 5 % |
| | Miglyol 812 | 7,5 % |
| | Gomme Q2 1401 | 4 % |
| B- | Glycérine | 5 % |
| | Triéthanolamine | 0,2 % |
| | Eau permutée | 61,3 % |
| C- | Isofol 16 | 7,5 % |
| | Rétinol | 0,5 % |

Cette crème appliquée en contour des yeux en cure de 2 à 5 fois par semaine a un effet de lissage des fines ridules du contour de l'oeil ou "pattes d'oie"

### Exemple 2 - Crème Antirides

| | | |
|---|---|---|
| A- | Abil EM-90 | 3 % |
| | Cyclométhicone D5 | 7 % |
| | Simagel Si 345 | 5 % |
| | Mygliol 812 | 11,5 % |
| | Gomme Q2-1401 | 3 % |
| B- | Glycérine | 5 % |
| | Triéthanolamine | 0,2 % |
| | NaCl | 0,7 % |
| | Eau permutée qsp | 100 |
| C- | Isofol 16 | 3 % |
| | Rétinol | 0,18 % |

Cette crème très douce et nourrissante est idéale en application quotidienne sur le visage, le soir, pour un effet anti-rides à long terme.

### Exemple 3 - Lait corporel anti-âge

| | | |
|---|---|---|
| A- | Abil EM-90 | 1 % |
| | Isolan Gi 34 | 5 % |
| | Paraffine liquide | 13 % |
| | Cyclométhicone D5 | 10 % |
| | Gomme Q2-1401 | 4 % |
| | Glycérine | 5 % |
| B- | Triéthanolamine | 0,2 % |
| | NaCl | 0,6 % |
| | Eau permutée qsp | 100 |
| C- | Diisopropyl adipate | 1 % |
| | Rétinol | 0,012 % |

Ce lait corporel souple est un excellent soin corporel anti-âge à condition de l'utiliser quotidiennement sur l'ensemble du corps. Après application, la peau est adoucie et lissée.

### Exemple 4 - Soin réparateur des lèvres

| | | |
|---|---|---|
| A- | Abil EM-90 | 3 % |
| | Simagel Si 345 | 5 % |
| | Cyclométhicone | 7 % |
| | Gomme Q2-1401 | 3 % |
| B- | AMP | 0,12 % |
| | NaCl | 0,7 % |
| | Glycérine | 5 % |
| | Eau permutée qsp | 100 |
| C- | Mygliol 812 | 11 % |
| | Rétinol | 0,9 % |

Cette crème compacte très glissante à l'application constitue un soin nourrissant et réparateur des lèvres abîmées et ridées.

Par ailleurs, il a été réalisé un test de stabilité dans lequel on a comparé une émulsion selon l'invention (exemple 1) et différentes formules dérivées de celles de l'exemple 1, (A,B,C,D,E,F).

### Composition A

| Phase grasse de l'exemple 1 à laquelle on ajoute : | |
|---|---|
| α-tocophérol | 0,1 % |
| acétate d'α-tocophérol | 1 % |
| palmitate d'ascorbyle | 0,2 % |

| + Phase aqueuse de l'exemple 1 dans laquelle on ajoute : | |
|---|---|
| Desquest 2046 * | 0,2 % |
| et dans laquelle l'eau permutée a été ajustée en conséquence. | |

| | |
|---|---|
| * Sel pentasodique de l'acide éthylène diamine tétra-méthylène phosphonique à 33 % tel que vendu par la Société Monsanto | |

### Composition B

| Phase grasse de l'exemple 1 à laquelle on ajoute : | |
|---|---|
| Palmitate d'ascorbyle | 0,2 % |
| [(ditertiobutyl-3,5 hydroxy-4) benzylidène]-3 camphre + Phase aqueuse de la composition A. | 0,5 % |

### Composition C

Phase grasse de l'exemple 1 + phase aqueuse de l'exemple 1 de laquelle on a retiré la triéthanolamine.

### Composition D

Phase grasse de la composition de l'exemple 1 + phase aqueuse de l'exemple 1 + phase C contenant le rétinol dissous dans 0,8 g de polysorbate 20 et dans 6,9 g de cyclohexadiméthylsiloxane.

### Composition E

Phase grasse de l'exemple 1 à laquelle on a ajouté 0,5% d'éthoxyquine pure (Raluquin de la Société Raschig) + phase aqueuse de l'exemple 1.

### Composition F

Composition de l'exemple 1 dans laquelle est incorporé après fabrication l'absorbeur d'oxygène isolé de l'émulsion.

Les tests de stabilité de ces compositions ont été effectués par mesure du pourcentage de dégradation après conservation pendant 1 mois ou 2 mois, soit à 4°C, soit à température ambiante, soit à 45°C. Les résultats sont donnés dans le tableau suivant:

| | 1 MOIS | | | 2 MOIS | | |
|---|---|---|---|---|---|---|
| Compositions | 4°C | Températ. ambiante | 45°C | 4°C | Températ. ambiante | 45°C |
| 1 | 0 | 0 | 19 | 6 | 10 | 33 |
| A | 2 | 5 | 25 | 4 | 12 | 35 |
| B | 0 | 1 | 27 | 2 | 18 | 37 |
| C | 0 | 8 | 44 | 4 | 34 | 72 |
| D | 0 | 1 | 40 | 0 | 14 | 68 |
| E | 0 | 0 | 9 | 0 | 0 | 27 |
| F | 0 | 1 | 6 | 1 | 16 | 18 |

Les résultats de ces tests montrent que l'émulsion selon l'invention (ex.1, F) présente une stabilité du rétinol comparable voire meilleure, à celle contenant un ou plusieurs antioxydants (A, B) comme décrit dans l'Art antérieur alors que des émulsions réalisées avec soit un rétinol dissous dans un solvant autre que ceux sélectionnés dans l'invention (D), soit un rétinol en présence d'une phase aqueuse à un pH inapproprié (C) présentent une moins bonne stabilité. Ce tableau montre la possibilité de stabiliser le rétinol dans des émulsions sans antioxydant, mais montre également que le choix d'un antioxydant particulier (Raluquin, E) introduit dans l'émulsion selon l'invention, peut améliorer la stabilité.

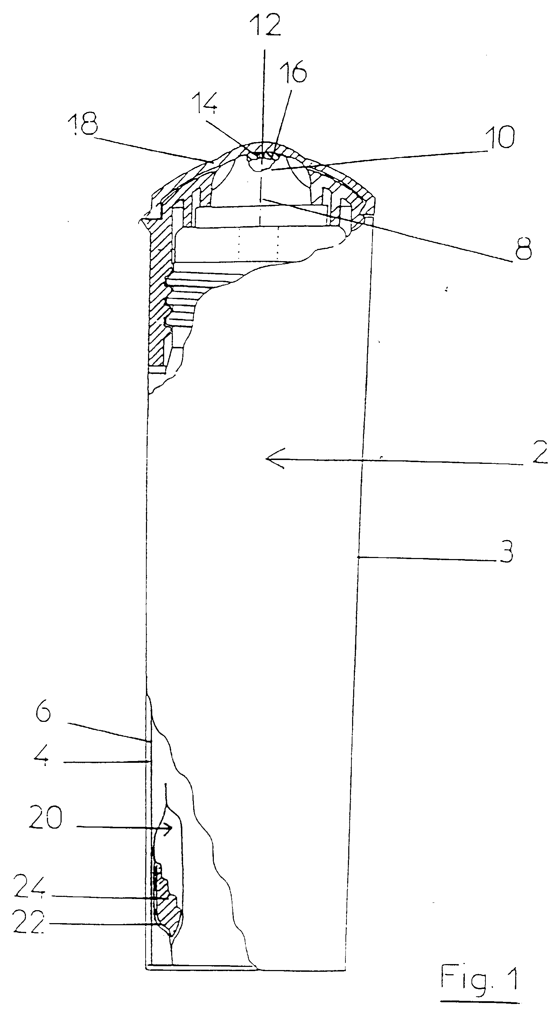

On décrit ci-après, en référence à la figure annexée (Fig. 1), un exemple de conditionnement compatible avec l'émulsion selon l'invention.

Le conditionnement (2) comporte un conteneur (3) dont les parois (4) sont constituées par tout matériau imperméable au gaz pouvant être fait, par exemple, d'un matériau thermoformé ou de tout autre matériau approprié à condition que la surface interne desdites parois, en contact avec l'émulsion (6), soit non métallique et que ce matériau soit imperméable aux rayons ultra-violets visibles.

Le conteneur (3) est surmonté d'un dispositif de distribution sans reprise d'air (8), comme par exemple celui décrit dans le document FR-A-2,666,308, constitué d'un dôme convexe vers l'extérieur (10) et d'au moins une fente (12) prévue au sommet du dôme.

Les parois (14, 16) de la fente (12) sont propres à venir en contact l'une contre l'autre pour réaliser une fermeture étanche au repos. L'ensemble du dispositif (8) est surmonté d'un capot hermétique (18) assurant une double protection de l'émulsion selon l'invention contenue dans le conditionnement, vis-à-vis de l'environnement.

Une poche (20) indépendante du conditionnement, dont les parois (22) sont formées d'une membrane perméable au gaz et imperméable au liquide, comme par exemple celle décrite dans le document FR-A-2,671,055, contient un absorbeur d'oxygène (24). L'absorbeur d'oxygène (24) encapsulé forme le dispositif de capture de l'oxygène noyé dans l'émulsion selon l'invention contenue dans le dispositif de conditionnement.

La membrane entourant le dispositif de capture de l'oxygène peut être réalisée en tout matériau susceptible de laisser passer l'oxygène tout en restant imperméable aux liquides en particulier aux liquides constituant l'émulsion selon l'invention et par exemple en l'un des matériaux choisis parmi le polyéthylène, le propylène et le polyéthylène-téréphtalate.

Au repos, les parois (14,16) du dôme (10) sont en contact étroit, assurant ainsi l'herméticité de la fente (12). En exerçant une pression sur le conditionnement (2), on transmet à l'émulsion contenue dans ce conditionnement une pression qui aura tendance à écarter les parois (14,16) permettant ainsi à l'émulsion de sortir du conditionnement. La pression étant relâchée, les parois (14,16) retrouvent leur position de repos et ferment de manière occlusive la fente (12).

## Revendications

1. Dispositif de conditionnement d'un produit, caractérisé par le fait qu'il est muni d'un dispositif de distribution tel que le produit n'est pas mis en contact avec l'environnement, que les parois le constituant sont imperméables aux gaz et à la lumière ultraviolette visible, leur surface en contact avec le produit étant non métallique, et qu'il contient en outre un dispositif de capture de l'oxygène.

2. Dispositif de conditionnement selon la revendication 1, caractérisé par le fait que le dispositif de distribution est constitué par un dispositif sans reprise d'air.

3. Dispositif de conditionnement selon la revendication 1 ou 2, caractérisé par le fait que ledit dispositif de capture de l'oxygène est isolé du produit par une membrane perméable au gaz et imperméable aux liquides.

4. Dispositif de conditionnement selon l'une quelconque des revendications précédentes, caractérisé par le fait que le produit est une émulsion eau dans huile contenant du rétinol, une phase grasse, une phase aqueuse et un émulsionnant de la phase aqueuse dans la phase grasse, la phase grasse contenant au moins un solvant organique du rétinol, liquide à température ambiante et le pH de la phase aqueuse étant basique de façon à stabiliser le rétinol au moins deux mois.

5. Dispositif de conditionnement selon la revendication précédente, caractérisé par le fait que l'émulsion est à usage cosmétique ou dermatologique.

6. Dispositif de conditionnement selon la revendication 4 ou 5, caractérisé en ce que l'émulsion contient du rétinol à raison de 0,001 % à 10 % du poids total de l'émulsion.

7. Dispositif de conditionnement selon l'une quelconque des revendications 4 à 6, caractérisé par le fait que le solvant organique représente de 0,01 % à 30 % du poids total de l'émulsion.

8. Dispositif de conditionnement selon l'une quelconque des revendications 4 à 7, caractérisé par le fait que le solvant organique est choisi parmi les alcools gras aliphatiques à chaîne ramifiée en C₁₆-C₂₀, les diesters d'acide dicarboxylique en C₆-C₁₄ et d'alcool isopropylique, les triglycérides d'acide gras en C₆-C₁₈ et leurs mélanges.

9. Dispositif de conditionnement selon l'une quelconque des revendications 4 à 8, caractérisé par le fait que l'émulsionnant est un émulsionnant siliconé.

10. Dispositif de conditionnement selon la revendication précédente, caractérisé par le fait que l'émulsionnant siliconé est présent en une proportion de 0,5 % à 10 % du poids total de l'émulsion.
